# EUROPÄISCHE PATENTSCHRIFT

(11) **EP 0 855 169 B1**
(45) Veröffentlichungstag und Bekanntmachung des Hinweises auf die Patenterteilung: **05.04.2000**
(21) Anmeldenummer: 98100694.3
(22) Anmeldetag: 16.01.1998
(51) Int. Cl.: A61B 17/70

(54) **Klemmanordnungen für orthopädischen Fixateur und Verwendungen derselben**
Clamp arrangements for orthopaedic fixator and applications thereof
Dispositifs de serrage pour fixateur externe et leurs utilisations

(30) Priorität: 23.01.1997 CH 14097
(43) Veröffentlichungstag der Anmeldung: 29.07.1998
(73) Patentinhaber: Hermann, Werner, 6312 Steinhausen (CH)
(72) Erfinder: Hermann, Werner, 6312 Steinhausen (CH)
(74) Vertreter: Hotz, Klaus, Dipl.-El.-Ing./ETH

(56) Entgegenhaltungen:
- US-A- 5 352 226

## Beschreibung

Die Erfindung betrifft Klemmanordnungen für orthopädische Fixateure nach Anspruch 1 bzw. **7** sowie einen Fixateur nach dem Anspruch **11** bzw. **12**.

Klemmanordnungen dieser Art werden in der Orthopädie und Traumatologie zu verschiedenen Zwecken als Bestandteile interner oder externer Fixateure verwendet. Beispielsweise beschreibt das **CH-Patentgesuch Nr. 00186/96** bzw. die entsprechende **EP-A-0 786 235**, welche nach der Hinterlegung der vorliegenden Anmeldung publiziert wurde, einen internen Fixateur, der bei krankhafter Veränderung wie Spondylose an Wirbelkörpern und Bandscheiben zur Anwendung gelangt, wobei die Stellung der Wirbelkörper korrigiert und die durch die Korrektur hergestellte relative Lage der betroffenen Wirbelkörper fixiert wird. Ferner beschreibt die **US-A-5,352,226** eine Anordnung, bei welcher ebenfalls ein Implantatstift in einem Klammkörper durch Zusammenspannung zweier Spannbacken festgehalten wird. Der Stift wird jedoch nicht unmittelbar im Klemmkörper gehalten, sondern es ist eine zwischen Implantatstift und Klemmkörper liegende spärische Buchse vorhanden. Diese Buchse erlaubt eine beschränkte Relativbewegung der Pedikelschrauben. In Fällen, in denen dies nicht erwünscht ist, ist diese Anordnung ungeeignet.

Grundsätzlich besteht ein Fixateur der eingangs genannten Art aus einem Implantatstift, der in einem Knochen befestigt wird. Als Implantatstifte werden im allgemeinen Stifte mit Gewinde, also Schrauben verwendet, die in eine Vorbohrung des Knochens eingeschraubt werden. Der ausserhalb des Knochens befindliche Teil des Implantatstiftes ragt durch eine erste Ausnehmung bzw. Bohrung eines Klemmkörpers. Im Rahmen der vorliegenden Beschreibung soll unter dem Begriff 'Klemmanordnung' der Klemmkörper und der darin befestigte Implantatstift verstanden werden. Der Klemmkörper besitzt eine zweite Bohrung, die quer zur ersten Bohrung gerichtet ist und in der eine Klemmschraube aufgenommen ist. Ein von der ersten Bohrung ausgehender, senkrecht zur Längsachse der Klemmschraube verlaufender Schlitz bildet am Klemmkörper zwei federnde Spannbacken. Der Schlitz ist so angeordnet, dass seine fiktive Verlängerung die Längsachse des Implantatstiftes enthält. Die Klemmschraube dient dazu, die Spannbacken zusammenzuspannen und dabei den Implantatstift im Klemmkörper zu fixieren. Der Kopf der Klemmschraube ist so ausgebildet, dass er eine Verbindungsstange aufnehmen kann, welche sich auch durch entsprechende Köpfe von Klemmschrauben eines oder mehrerer weiterer Fixateure erstreckt, wobei die Verbindungsstange je nach Bedarf gerade oder gebogen sein kann. Bei internen Fixateuren befinden sich alle oben beschriebenen Bauteile innerhalb des Körpers des Implantatträgers, während bei externen Fixateuren lediglich ein Teil des Implantatstiftes im Körper des Implantatträgers angeordnet ist, während der Rest des Implantatstiftes aus dem Körper hinausragt, so dass der Klemmkörper und die Verbindungsstange sich ausserhalb des Körpers des Implantatträgers befinden.

Bei Bewegungen des Implantatträgers bewegen sich der Implantatstift und der Klemmkörper ebenfalls, wobei es auch zu Relativbewegungen zwischen dem Implantatstift und dem Klemmkörper kommt. Unabhängig davon, ob es sich um lineare Bewegungen in der Richtung der Achse des Implantatstiftes, um Rotationen des Implantatstiftes oder um Verbiegungen des Implantatstiftes handelt, sind diese Relativbewegungen stets nur Mikrobewegungen. Die bei den Relativbewegungen zwischen dem Implantatstift und dem Klemmkörper übertragenen Kräfte haben nicht nur reibungsbedingte mechanische Abnützungserscheinungen der Oberfläche sondern - da es sich um Wechselbeanspruchungen handelt - vor allem eine Ermüdung des Materials zur Folge, welche die Lebensdauer der Klemmanordnung negativ beeinflusst. Diese unerwünschte Erscheinung tritt nicht nur in den Querschnitten, in welchen der Implantatstift aus dem Klemmkörper austritt, sondern an der gesamten Berührungsfläche zwischen Implantatstift und Klemmkörper auf. Insbesondere hat die Materialermüdung die Bildung von feinen Rissen zur Folge, was sich besonders am Implantatstift als dem mechanisch schwächeren Teil ungünstig auswirkt. Treten nämlich im Lauf der Zeit zusätzlich infolge besonderer Bewegungen des Implantatträgers Spannungsspitzen auf, so können sich dadurch von den feinen Ermüdungsrissen ausgehende grössere Risse ausbilden, aus denen sich letztlich Gewaltbrüchen entwickeln können. Ungünstig ist es auch, dass, wie schon erwähnt, durch die Reibungskräfte zwischen der Mantelfläche des Implantatstifts und der Wandung der Ausnehmung des Klemmkörpers Abriebpartikel entstehen, welche ebenfalls ein Zeichen für unerwünschte Abnützungserscheinungen sind und im Fall von internen Fixateuren ausserdem zur Bildung von Metallose führen können; zwar werden Implantate generell aus Werkstoffen gefertigt, die physiologisch gut verträglich sind; dies gilt jedoch nur für Teile mit makroskopischen Abmessungen, während mikroskopische Teile wie eben beispielsweise Abriebpartikel aus gleichen Werkstoffen physiologisch unverträglich sein und die erwähnte Metallose zur Folge haben können.

Die Aufgabe der Erfindung wird somit darin gesehen, die vorbekannten Klemmanordnungen so zu modifizieren, dass die erwähnten Nachteile nicht auftreten.

Dies wird erfindungsgemäss durch die Merkmale des Patentanspruch 1 gelöst. Vorteilhafte Weiterbildungen und bevorzugte Ausführungsbeispiele der erfindungsgemässen Klemmanordnung werden durch die Patentansprüche 2 - 6 definiert.

Demzufolge ist vorgesehen, dass entweder die Aussenfläche des Implantatstiftes oder die Innenfläche der Ausnehmung rückspringende Bereiche aufweisen, wodurch die restlichen, auf der ursprünglich zylindrischen Fläche liegenden Bereiche gewissermassen als Vorsprünge erscheinen, deren Kulminationsbereiche die jeweils gegenüberliegenden zylindrischen Flächenabschnitte berühren. Die Führung des Implantatstiftes erfolgt dabei immer noch über die gesamte Länge der Ausnehmung des Klemmkörpers. Es sind jedoch Mikrobewegungen, insbesondere in axialer Richtung, mit verminderter Reibwirkung möglich; dadurch wird die Abnützung vermindert. Der Grund für eine verminderte Bildung von Ermüdungsrissen ist darin zu sehen, dass die Vorsprünge der einen Fläche, welche die andere Fläche berühren, eine gewisse Elastizität aufweisen und sich unter den bei relativbewegung wirkenden Kräften um ein weniges elastisch deformieren, so dass lokal die Relativbewegungen und damit das Auftreten von wechselbeanpruchungsbedingten Ermüdungserscheinungen im Material verhindert oder jedenfalls stark reduziert wird, was die Folge hat, dass die Lebensdauer der Klemmanordnung erhöht wird. Gleichzeitig wird die Bildung von Abrieb pro Zeit vermindert. Wenn die Verminderung von Abrieb auch generell angestrebt wird, so ist sie besonders notwendig bei den erfindungsgemässen Fixateuren mit erhöhter Lebensdauer. Die Abriebmenge nimmt nämlich - bei sonst gleicher Ausbildung der Bauteile - mit der Verwendungszeit zu. Bei Implantaten mit hoher Lebensdauer ist es daher vorteilhaft, wenn die zeitlich anfallende Abriebmenge klein ist, so dass die insgesamt während der Verwendung des Implantats anfallende Abriebmenge nicht zu gross wird, so dass eine erhöhte Lebensdauer der Implantate die Metallosegefahr nicht erhöht.

Die erfindungsgemässe Klemmanordnung wird im allgemeinen so ausgebildet, dass die die rückspringenden Bereiche aufweisende Fläche die Innenfläche der Bohrung bzw. Ausnehmung ist, während der Implantatstift - mit Ausnahme eines eventuellen Gewindes zum Einschrauben in einen Knochen - zylindrisch ausgebildet ist. Eine solche Ausbildung wird häufig bevorzugt, da durch das Erzeugen der rückspringenden Bereiche eine Schwächung entsteht, und eine solche kann im allgemeinen eher beim Klemmkörper als beim Implantatstift in Kauf genommen werden.

Die Anordnung der rückspringenden Bereiche an der Aussenfläche des Implantatstifts ist jedoch auch möglich und wird gelegentlich, insbesondere bei komplizierter Formgebung der rückspringenden Flächen, bevorzugt, da im allgemeinen die Bearbeitung von Aussenflächen einfacher ist als die Bearbeitung von Innenflächen.

Die rückspringenden Bereiche können in einfacher Weise dadurch erzeugt werden, dass die entsprechende zylindrische Fläche mit einer schraubenlinienförmigen Ausnehmung, also im Prinzip mit einem Gewinde, versehen wird.

Statt durch eine schraubenlinienförmige Ausnehmung können die rückspringenden Bereiche auch durch mehrere Rillen bzw. Nuten gebildet werden, die vorzugsweise senkrecht zur Längsachse der ersten Ausnehmung bzw. des Implantatstiftes verlaufen.

Unabhängig davon, ob die rückspringenden Bereichen durch eine Schraubenlinie oder durch mehrere Nuten gebildet werden, können sie im einzelnen so ausgebildet sein, dass der Querschnitt der Schraubenlinie bzw. der Nuten gerundet, trapezartig, rechteckig oder schwalbenschwanzähnlich ist; die rückspringenden Bereiche und die sie trennenden Bereiche der ursprünglichen zylindrischen Fläche bilden dann in einem die Längsachse der Ausnehmung bzw. des Implantatstiftes enthaltenden Schnitt eine Folge von Wellen, Trapezen, Rechtecken oder Schwalbenschwänzen, wobei vorzugsweise durch geeignete Rundungen an allen Übergängen Kerbwirkungen vermieden werden.

Die weiter oben erwähnten Relativbewegungen zwischen dem Implantatstift und dem Klemmkörper, die bei Bewegungen des Implantatträgers auftreten, können besonders bei Biegungen des Implantatstiftes unangenehme Folgen haben. Es liegt auf der Hand, dass die biegungsbedingten Deformationen des Implantatstiftes grösser sind als diejenigen des Klemmstückes. Der in der Ausnehmung aufgenommene Implantatstift wird sich unter Biegebeanspruchung innerhalb der Ausnehmung praktisch nicht verbiegen, da dabei ja auch das Klemmstück im Bereich der Ausnehmung entsprechend deformiert werden müsste, was infolge des grösseren Biegewiderstandes des Klemmstückes nur sehr beschränkt der Fall ist. Hingegen unterliegt der Implantatstift an den beiden Austrittsquerschnitten, in welchen er aus dem Klemmkörper austritt, einer erhöhten Kerbwirkung durch die Berandung der Ausnehmung des Klemmkörpers, welche natürlich ebenfalls als Wechselbelastung auftritt und Materialermüdung zur Folge hat.

Es ist somit ebenfalls Aufgabe der Erfindung, einen Klemmkörper der vorbekannten Art vorzuschlagen, bei welchen die Beanspruchung des Implantatstiftes insbesondere in den Austrittsquerschnitten reduziert wird.

Diese weitere Aufgabe wird durch die Merkmale des Patentanspruchs **7** gelöst. Die Patentansprüche **8 - 10** definieren entsprechende Ausführungsbeispiele.

Somit werden die Aussenflächen des Implantatstiftes im Bereich seiner beiden Austrittsquerschnitte oder die Innenfläche der zur Aufnahme des Implantatstiftes bestimmten Ausnehmung bzw. Bohrung im Bereich ihrer Endquerschnitte mit Entlastungsausnehmungen versehen. Dadurch verhindert man, dass bei Biegung des Implantatstiftes der letztere mit einem kleinen Sektor seines Umfanges an einen kerbenden Kantenbereich der Ausnehmungen gepresst wird. Auf diese Weise kann die Entstehung von feinsten Anrissen vermieden oder reduziert werden. Allerdings wird die Führungslänge des Implantatstiftes im Klemmkörper reduziert und es tritt keine Verminderung der Beanspruchung bei axialen und drehenden Relativbewegungen ein.

Es sei noch erwähnt, dass die Möglichkeit besteht, Entlastungsausnehmungen sowohl am Implantatstift wie auch am Klemmkörper anzubringen, doch ist der dadurch zusätzlich erreichbare positive Effekt gering.

Bei einer vorteilhaften Ausbildung der Klemmanordnung werden zur Erzeugung der Entlastungsausnehmungen die kreisförmigen Kanten an den Übergängen von der Ausnehmung zu den Aussenflächen des Klemmkörpers gerundet oder gebrochen, während der Implantatstift nicht weiter bearbeitet wird. Durch diese Massnahme erreicht man, dass bei der Biegung des Implantatstiftes entweder keine Berührung der Kantenbereiche auftritt oder keine Starke Anpressung an kerbende Sektoren der Kantenbereiche vorkommt. Dadurch verringert sich die Entstehung der feinen Anrisse, was zur Folge hat, dass auch bei gelegentlichen Spannungsspitzen die Gefahr eines Bruches stark gemindert wird.

Bei einer anderen Ausführungsart der Klemmanordnung werden die Entlastungsausnehmungen am Implantatstift vorgesehen, und zwar in Form von umlaufenden Nuten an denjenigen Bereichen des letzteren, die Austrittsquerschnitte enthalten, welche je nach Einstellung des Fixateurs innerhalb eines gewissen Bereiches des Implantatstiftes liegen können. Diese Ausführung hat allerdings die an sich unerwünschte Folge, dass der Implantatstift infolge der Verminderung seines Durchmessers an zwei Bereichen geschwächt wird. Die Schwächung kann aber stark vermindert werden, wenn die Nuten abgerundete Übergänge aufweisen. In Fällen, in denen der Implantatstift eine Schraube ist, ist die erwähnte Schwächung ohnehin von geringem Belang, solange der Durchmesser des Implantatstiftes im Nutgrund nicht kleiner ist als der Innendurchmesser des Gewindes. Eine solche Lösung kann allerdings auch bedeuten, dass der Durchmesser des Implantatstiftes erhöht werden muss.

Die Anordnung besonderer Entlastungsausnehmungen erübrigt sich bei den erfindungsgemässen Klemmanordnungen, bei welchen der Implantatstift oder die Ausnehmung des Klemmkörpers rückspringende Bereiche aufweisen; die zur Bildung der rückspringenden Bereiche dienende bildende Schraubenlinie bzw. die entsprechenden Nuten oder Rillen wirken bei geeigneter Anordnung und Ausbildung dann selbst als Entlastungsausnehmungen im Bereich der Austrittsquerschnitte.

Schliesslich ist es Aufgabe der Erfindung, entsprechende Fixateure vorzuschlagen.

Solche Fixateure weisen die erfindungsgemässen Klemmanordnungen auf und lassen sich in Kombination mit weiteren Fixateuren sowohl intern wie auch extern verwenden.

Im folgenden wird die Erfindung anhand mehrerer Ausführungsbeispiele und mit Bezug auf die Zeichnung näher erläutert. Es zeigen:
- **Fig.1**: eine Seitenansicht eines Fixateurs nach dem Stand der Technik mit zwei Implantatstiften;
- **Fig.2**: einen Schnitt gemäss der Linie II-II in der **Fig.1**,
- **Fig.3**: einen Schnitt durch einen Klemmkörper nach dem Stand der Technik;
- **Fig.4**: einen Schnitt durch eine Spannscheibe nach dem Stand der Technik;
- **Fig.5**: einen Teilschnitt durch eine Klemmschraube des Klemmkörpers nach dem Stand der Technik;
- **Fig.6**: einen Schnitt und eine Draufsicht durch beziehungsweise auf die Spannscheibe in einer anderen Ausführung, nach dem Stand der Technik,
- **Fig.7**: eine Ansicht einer Klemmschraube, in einer anderen Ausführung, nach dem Stand der Technik,
- **Fig.8**: einen Schnitt durch ein einen Klemmkörper mit einem Implantatstift, einer Klemmschraube, einer Spannscheibe und einer gebogenen Verbindungsstange in grösserem Massstab, nach dem Stand der Technik;
- **Fig.9**: einen Schnitt gemäss der Linie IX-IX in der **Fig.2** in grösserem Massstab;
- **Fig.10**: eine Rückansicht eines Teiles einer Wirbelsäule mit zwei parallelen Implantateinrichtungen mit je vier Klemmkörpern, nach dem Stand der Technik;
- **Fig. 11**: einen Klemmkörper nach der Erfindung, mit rückspringenden Berei chen an der Innenfläche der für den Implantatstift bestimmten Aus nehmung, in einer ersten Ausführung;
- **Fig. 12**: einen Klemmkörper nach der Erfindung, mit rückspringenden Berei chen an der Innenfläche der für den Implantatstift bestimmten Aus nehmung, in einer zweiten Ausführung;
- **Fig. 13**: eine im Schnitt gewellte Kontur der für den Implantatstift bestimmten Ausnehmung, ausschnittweise;
- **Fig. 14**: eine im Schnitt eine Folge von Trapezen bildende Kontur der für den Implantatstift bestimmten Ausnehmung;
- **Fig. 15**: eine im Schnitt eine Folge von Rechtecken bildende Kontur der für den Implantatstift bestimmten Ausnehmung;
- **Fig. 16**: eine im Schnitt eine Folge von Schwalbenschwänzen bildende Kontur der für den Implantatstift bestimmten Ausnehmung;
- **Fig. 17**: eine erfindungsgemässen Klemmanordnung, mit einem Klemmkörper und mit einem Implantatstift, mit Entlastungsausnehmungen am Klemmkörper, ausschnittweise; und
- **Fig. 18**: eine erfindungsgemässe Klemmanordnung mit einem Implantatstift, mit Entlastungsausnehmungen am Implantatstift, ausschnittweise.

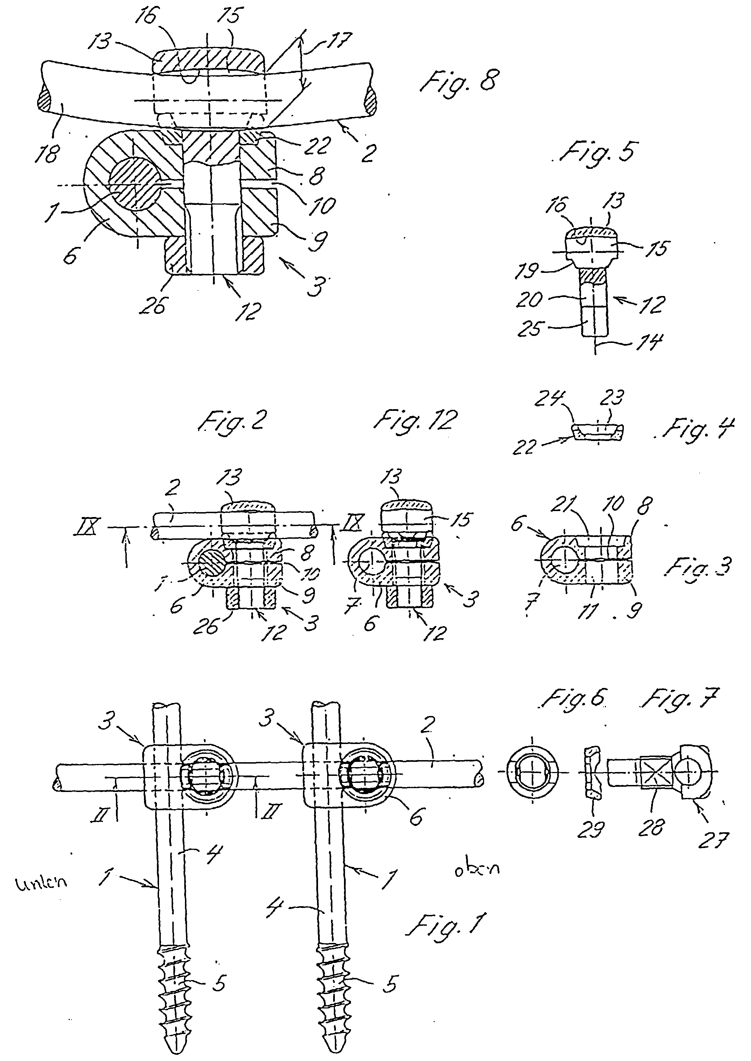

Die **Fig. 1 - 10** betreffen einen Stand der Technik, wie er durch die nachveröffentliche EP-**A-0 786 235** dokumentiert ist.

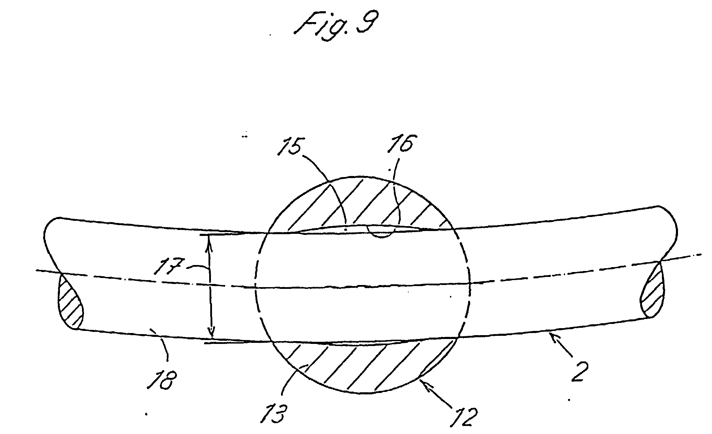

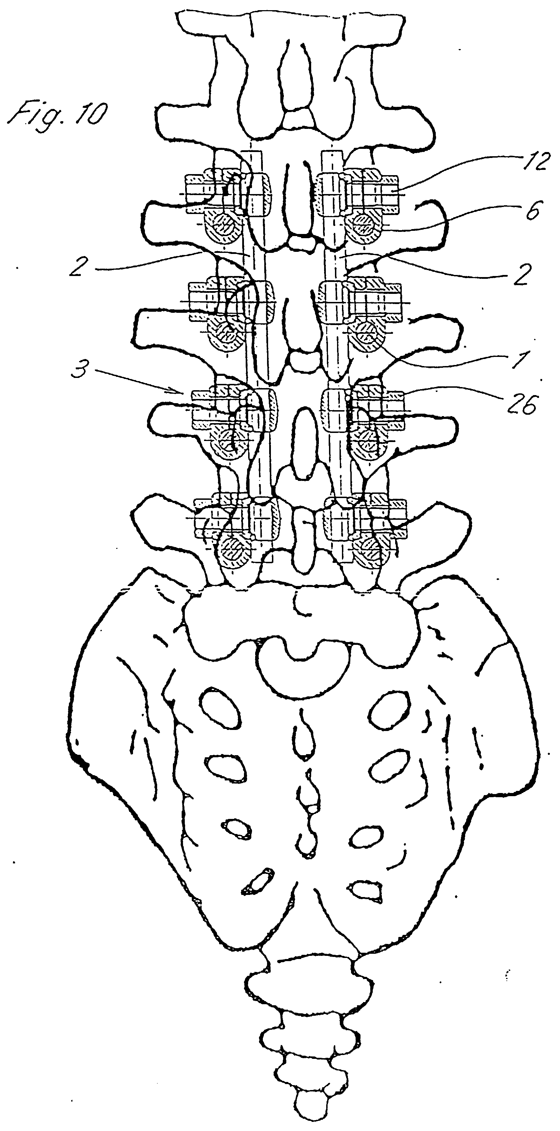

Dabei sind mit 1 Implantatstifte bezeichnet, welche gemäss **Fig. 1** bis **Fig. 10** als Pedikelschrauben ausgebildet sind, die über eine Verbindungsstange **2** und Verbindungsstücke **3** miteinander verbunden sind. Die Pedikelschrauben weisen einen Schaft **4** und ein Gewindeteil **5** auf, mit welchem sie in Wirbelkörper **30** einschraubbar sind, wie dies in **Fig. 10** dargestellt ist. Bei einer solchen parallelen Anordnung kann es sich zur Vermeidung von Vorspannungen im Wirbelkörper als günstig erweisen, Pedikelschrauben mit entgegengesetzten Gewinden zu verwenden. Die Verbindungsstücke **3** bestehen aus einem Klemmkörper **6**, der eine erste Ausnehmung bzw. Bohrung **7** für die Aufnahme einer Pedikelschraube **1** und einen mit der ersten Bohrung **7** verbundenen, zwei federnde Spannbacken **8,9** bildenden Schlitz **10** aufweist. In den Spannbacken **8,9** ist eine die erste Bohrung **7** kreuzende, senkrecht zum Schlitz **10** verlaufende zweite Bohrung **11** für die Aufnahme einer Klemmschraube **12** des Verbindungsstückes **3** vorgesehen. In einem Kopf **13** der Klemmschraube **12** ist eine senkrecht zur Längsachse **14** der Klemmschraube **12** verlaufende Bohrung **15** für die Führung und Festhaltung der Verbindungsstange **2** angeordnet. Die Oberfläche **16** der Bohrung **15** ist nach innen und nach aussen gewölbt, wobei der Durchmesser **17** der Bohrung **15** an ihren Enden annähernd dem Durchmesser der Verbindungsstange **2** entspricht und die Wölbung beispielsweise kreis- oder ellipsenförmig ist. In der derartig gestalteten Bohrung **15** kann sowohl ein gerader als auch ein gebogener Teil **18** einer individuell an die Krümmung der Wirbelsäule angepassten Verbindungsstange **2** geführt und gehalten werden, wobei die Verbindungsstange **2** an beiden Enden der Bohrung **15** linienförmig aufliegt, wie dies in den **Fig. 8** und **9** dargestellt ist. Ein Übergang **19** zwischen dem Kopf **13** und einem Schaft **20** der Klemmschraube **12** ist konisch ausgebildet, wobei die Bohrung **15** teilweise im konischen Übergang **19** verläuft.

In der einen Spannbacke **8** des Klemmkörpers **6** ist konzentrisch zur zweiten Bohrung **11** eine konische Vertiefung **21** vorgesehen, in welcher eine entsprechend geformte Spannscheibe **22** angeordnet ist. Die Spannscheibe **22** weist eine dem konischen Übergang **19** der Klemmschraube **12** angepasste konische Vertiefung **23** auf, wobei ein Rand **24** gebildet wird, in welchem ein Teil der Bohrung **15** verläuft.

Beim Fixieren des Implantats werden durch Verschrauben eines Gewindes **25** der Klemmschraube **12** mit einer Mutter **26** sowohl die Pedikelschrauben **1** als auch die Verbindungsstange **2** gleichzeitig festgeklemmt.

Gemäss der Ausführung nach **Fig. 6** und **7** weist die Klemmschraube **27** zwei parallel verlaufende Abflachungen **28** auf, die mit entsprechend geformten, nicht näher dargestellten Abflachungen in der Spannscheibe **29** - mit Langloch passend zu Abflachung **28** - und der zweiten Bohrung **11** korrespondieren. Mit dieser Massnahme soll ein Verdrehen der Klemmschraube **27** verhindert werden beziehungsweise in gelöstem Zustand das Mitdrehen der Spannscheibe garantieren, damit in jeder Lage sichergestellt ist, dass die Verbindungsstange nicht aus den Vertiefungen rutscht und damit in der Lage wäre sich zu Verdrehen.

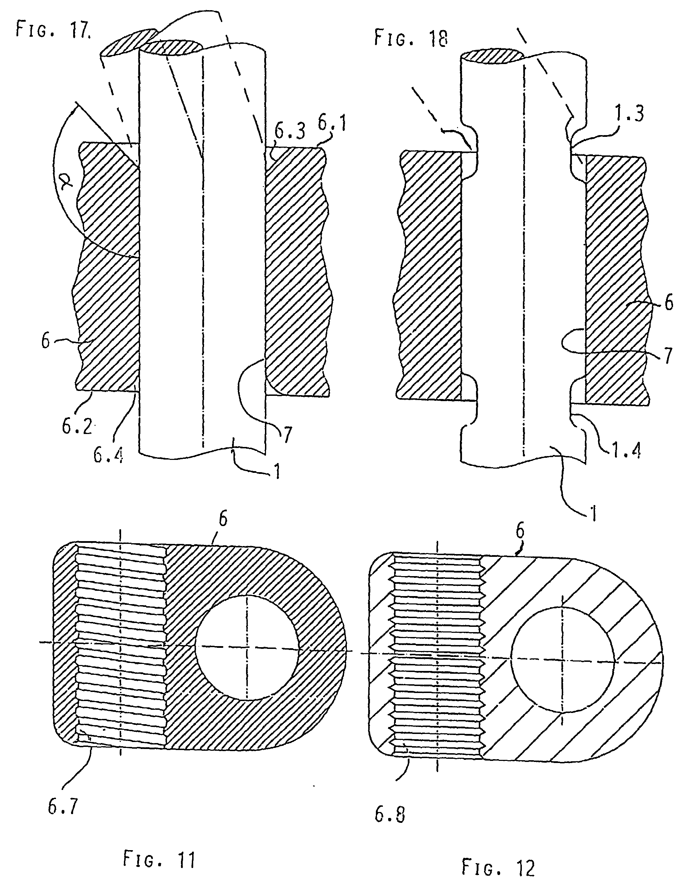

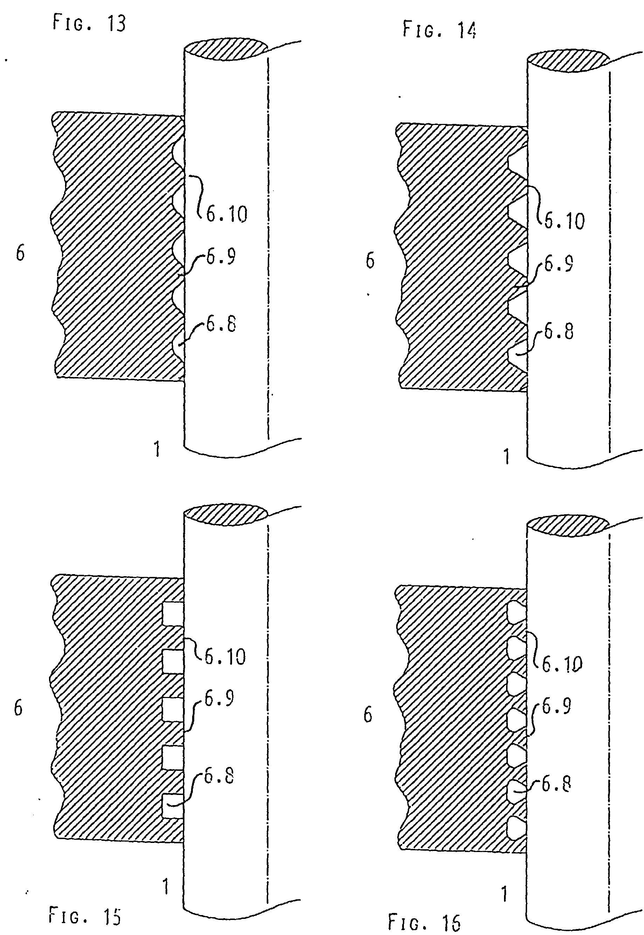

Im folgenden werden mit Bezug auf die **Fig. 11 - 18** die erfindungsgemässen Klemmanordnungen beschrieben.

**Fig. 11** zeigt einen Klemmkörper **6** mit rückspringenden Bereichen an der ursprünglich zylindrischen Innenfläche der Ausnehmung bzw. Bohrung **7**, welche durch eine schraubenlinienförmige Ausnehmung **6.7** gebildet sind; genau genommen handelt es sich um einen einzigen, zusammenhängenden rückspringenden Bereich. In ähnlicher Weise sind beim Klemmstück **6** gemäss **Fig. 12** rückspringende Bereiche durch umlaufende Nuten oder Rillen **6.8** gebildet.

Der bzw. die rückspringenden Bereiche in Form einer Schraubenlinie bzw. in Form mehrerer Rillen oder Nuten können statt am Klemmstück **6** auch in nicht dargestellter Weise am Implantatstift **1** angeordnet sein.

Die **Fig. 13 - 16** zeigen verschiedene Ausbildungsmöglichkeiten für die Konturen von rillenartigen Ausnehmungen **6.8**, welche die rückspringenden Bereiche der Ausnehmung **7** bei einem Klemmkörper gemäss **Fig. 12** bilden.

Die gegenseitige Berührung zwischen dem zylindrischen Implantatstift **1** und der die Ausnehmung **7** begrenzenden Fläche ist durch das Vorhandensein der rückspringenden Bereiche **6.8** auf die Kulminationsflächen **6.10** der ins Innere der Bohrung **7** ragenden Vorsprünge **6.9**. Allfällige Kräfte, die zwischen dem Implantatstift **1** und dem Klemmkörper **6** übertragen werden, können somit nur via diese Kulminationsflächen **6.10** stattfinden. Die Vorsprünge **6.9** werden durch solche Kräfte minimal elastisch deformiert, wodurch die Entstehung von Materialermüdung und Abrieb vermindert wird.

**Fig. 17** zeigt den Klemmkörper **6** mit der ersten Bohrung bzw. Ausnehmung **7** und den Implantatstift **1**. Der Klemmkörper 6 weist beim Übergang von der Ausnehmung **7** zur seiner oben liegenden Aussenfläche **6.1** eine Entlastungsausnehmung in Form einer gebrochenen umlaufenden Kante **6.3** auf, die über eine kleine Rundung in die Wandung der Ausnehmung **7** übergeht. Auch beim Übergang von der Wandung der Ausnehmung **7** zur unten liegenden Aussenfläche **6.2** ist der Klemmkörper **6** mit einer Entlastungsausnehmung versehen, welche hier die Form einer Abrundung **6.4** besitzt.

Im Ausführungsbeispiel gemäss **Fig. 18** ist anstelle des Klemmkörpers **6** der Implantatstift **1** im Bereich seines oberen Austrittsquerschnitts **1.1** und seines unteren Austrittsquerschnitts **1.2** mit je einer Entlastungsausnehmung in Form einer umlaufenden Nut oder Rille **1.3** bzw. **1.4** versehen.

Sowohl in **Fig. 17** wie auch in **Fig. 18** ist mit gestrichelten Linien angedeutet, welche Lage ein oberhalb des Klemmkörpers 6 abgebogener Implantatstift **1** - allerdings bei starker Übertreibung des Biegewinkels - einnehmen würde. Man erkennt deutlich, dass bei einer Anordnung gemäss **Fig. 18** infolge der Entlastungsausnehmungen **1.3, 1.4** weder eine Pressung noch eine kerbende Wirkung von der Kante **6.0** des Klemmkörpers **6** auf den Implantatstift 1 ausgeübt werden kann. Bei einer Anordnung gemäss **Fig. 17** oben ist zwar - wenn keine zusätzliche Abrundung vorgesehen wird - eine Kerbwirkung vorhanden, die aber dadurch entschärft wird, dass gegebenenfalls die kerbende Kante **6.9** nicht einen rechten Winkel bildet, wie es ohne Kantenbrechung bzw. Abschrägung **6.3** der Fall wäre, sondern einen stumpfen Winkel α. Bei einer Anordnung gemäss **Fig. 17** unten ist eine kerbende Wirkung infolge der Abrundung 6.4 ausgeschlossen.

Die Fig. **13 - 16** zeigen, dass bei geeigneter Anordnung und Dimensionierung der die rückspringenden Bereiche **6.8** bildenden Rillen die letzteren auch als Entlastungsausnehmungen im Bereich der Endquerschnitte **6.1, 6.2** des Klemmkörpers 6 dienen.

Es sei noch erwähnt, dass die Zahl der Lastwechsel bis zum Bruch, die ein Massstab für die Lebensdauer sind, für Fixateure mit Klemmanordnungen nach der Erfindung im Bereich des Doppelten von Fixateuren mit Klemmanordnungen gemäss der weiter oben mehrfach erwähnten **EP-A-0 786 235** liegen, wie dies auch in einer Testanordnung festgestellt wurde. Trotz der erhöhten Lebensdauer nimmt dabei die Gesamtmenge des Abriebes nicht zu, was bedeutet, dass eine zeitlich verminderte Abriebmenge anfällt.

Die oben beschriebenen Klemmanordnungen stellen nur eine kleine Auswahl der im Rahmen der Erfindung möglichen Klemmanordnungen dar.

## Patentansprüche

1. Klemmanordnung **(1, 6)** für orthopädischen Fixateur, mit einem in einem Knochen fixierbaren Implantatstift **(1)** und mit einem Klemmkörper **(6)**, der eine Ausnehmung **(7)** aufweist, deren Begrenzungsfläche zwei gegenüberliegende Aussenflächen **(6.1, 6.2)** des Klemmkörpers **(6)** verbindet und den Implantatstift **(1)** unmittelbar aufnimmt, welcher Klemmkörper einen von der Ausnehmung **(7)** ausgehenden Schlitz aufweist, dessen gedachte Verlängerung die Längsachse der Ausnehmung **(7)** enthält und durch welchen Schlitz am Klemmkörper **(6)** zwei federnde Spannbacken **(8, 9)** gebildet sind, bei deren Zusammenspannung der Implantatstift **(1)** im Klemmkörper **(6)** befestigt ist, wobei die eine der einander zugewandten Flächen der Ausnehmung **(7)** des Klemmkörpers **(6)** und des Implantatstiftes **(1)** rückspringende Bereiche **(6.7, 6.8)** aufweist.

2. Klemmanordnung **(1, 6)** nach Patentanspruch **1**,
**dadurch gekennzeichnet**,
dass die die rückspringenden Bereiche aufweisende Fläche die Wandung der Ausnehmung **(7)** des Klemmkörpers **(6)** ist.

3. Klemmanordnung **(1, 6)** nach Patentanspruch **1**,
**dadurch gekennzeichnet**,
dass die die rückspringenden Bereiche aufweisende Fläche die Aussenfläche des Implantatstiftes **(1)** ist.

4. Klemmanordnung **(1, 6)** nach Patentanspruch 1,
**dadurch gekennzeichnet,**
dass die rückspringenden Bereiche durch eine schraubenlinienartige Ausnehmung **(6.7)** gebildet sind.

5. Klemmanordnung **(1, 6)** nach Patentanspruch **1**,
**dadurch gekennzeichnet,**
dass die rückspringenden Bereiche durch mehrere vorzugsweise senkrecht zur Längsachse der Ausnehmung **(7)** bzw. des Implantatstiftes **(1)** verlaufende Rillen **(6.8)** gebildet sind.

6. Klemmanordnung **(1, 6)** nach Patentanspruch **4** oder **5**,
**dadurch gekennzeichnet,**
dass jeder rückspringende Bereich in einem Schnitt durch die Längsachse der Ausnehmung **(7)** bzw. des Implantatstiftes **(1)** eine wellenlinienartige, trapezartige, rechteckige oder schwalbenschwanzähnliche Kontur, ggfs. mit abgerundeten Kanten und Ecken, bildet.

7. Klemmanordnung **(1, 6)** für orthopädischen Fixateur, mit einem in einem Knochen fixierbaren Implantatstift **(1)** und mit einem Klemmkörper **(6)**, welcher eine Ausnehmung **(7)** aufweist, deren Begrenzungsfläche zwei gegenüberliegende Aussenflächen **(6.1, 6.2)** des Klemmkörpers **(6)** verbindet und den Implantatstift **(1)** unmittelbar aufnimmt, und welcher Klemmkörper **(6)** einen von der Ausnehmung **(7)** ausgehenden Schlitz aufweist, dessen gedachte Verlängerung die Längsachse der Ausnehmung **(7)** enthält und durch welchen am Klemmkörper **(6)** zwei federnde Spannbacken **(8, 9)** gebildet sind, bei deren Zusammenspannung der Implantatstift **(1)** im Klemmkörper **(6)** befestigt ist, wobei der Klemmkörper **(7)** oder/und der Implantatstift **(1)** im Bereich der Austrittsquerschnitte **(1.1, 1.2)** des Implantatstiftes **(1)** aus dem Klemmkörper **(6)** Entlastungsausnehmungen **(6.3, 6.4, 1.3, 1.4)** aufweisen, um bei Biegungen des Implantatstiftes **(1)** eine vom Klemmkörper **(6)** auf den Implantatstift **(1)** ausgeübte Kerb- und Presswirkung zu verhindern.

8. Klemmanordnung **(1, 6)** nach Patentanspruch **7**,
**dadurch gekennzeichnet,**
die Entlastungsausnehmungen am Klemmkörper **(6)** angeordnet und durch gebrochene oder abgerundete Kanten **(6.3, 6.4)** am Übergang zwischen den Aussenflächen **(6.1, 6.2)** und der Ausnehmung **(7)** des Klemmkörpers **(6)** gebildet sind.

9. Klemmanordnung **(1, 6)** nach Patentanspruch **8**,
**dadurch gekennzeichnet,**
dass die Entlastungsausnehmungen an Implantatstift **(1)** angeordnet und durch je eine umlaufende Nut **(1.3, 1.4)**, die vorzugsweise gerundete Kanten und Ecken besitzt, gebildet sind.

10. Klemmanordnung **(1, 6)** nach den Patentansprüchen **1** und **7**,
**dadurch gekennzeichnet**,
dass die Entlastungsausnehmungen durch die in der Umgebung der Austrittsquerschnitte angeordneten rückspringenden Bereiche gebildet sind.

11. Interner Fixateur mit einer Klemmanordnung **(1, 6)** nach mindestens einem der Patentansprüche **1 - 10**.

12. Externer Fixateur mit einer Klemmanordnung **(1, 6)** nach mindestens einem der Patentansprüche **1 - 10**.

## Claims

1. Clamp arrangement (1, 6) for an orthopaedic fixator, with an implant pin (1) adapted to be fixed in a bone, and with a clamp body (6) having a recess (7) whose boundary surface connects two opposing external surfaces (6.1, 6.2) of the clamp body (6) and directly receives the implant pin (1), said clamp body (6) incorporating a slot which starts from the recess (7) and the imaginary extension of which contains the longitudinal axis of the recess (7) and which on the clamp body (6) forms two resilient clamping jaws (8, 9) which when clamped together secure the implant pin (1) in the clamp body (6), one of the mutually facing surfaces of the recess (7) of the clamp body (6) and of the implant pin (1) incorporating recessed areas (6.7, 6.8).

2. Clamp arrangement (1, 6) according to claim 1,
characterised in that
the surface incorporating the recessed areas is the wall of the recess (7) of the clamp body (6).

3. Clamp arrangement (1, 6) according to claim 1,
characterised in that
the surface incorporating the recessed areas is the external surface of the implant pin (1).

4. Clamp arrangement (1, 6) according to claim 1,
characterised in that
the recessed areas are formed by a helically shaped recess (6.7).

5. Clamp arrangement (1, 6) according to claim 1,
characterised in that
the recessed areas are formed by a plurality of channels (6.8) which preferably run perpendicular to the longitudinal axis of the recess (7) and of the implant pin (1).

6. Clamp arrangement (1, 6) according to claim 4 or 5,
characterised in that
seen in cross-section through the longitudinal axis of the recess (7) and of the implant pin (1), each recessed area forms a contour with the shape of a wavy line, trapezium, rectangle or dovetail, with rounded edges and corners if appropriate.

7. Clamp arrangement (1, 6) for an orthopaedic fixator, with an implant pin (1) adapted to be fixed in a bone, and with a clamp body (6) having a recess (7) whose boundary surface connects two opposing external surfaces (6.1, 6.2) of the clamp body (6) and directly receives the implant pin (1), and which clamp body (6) incorporates a slot which starts from the recess (7), the imaginary extension of which slot contains the longitudinal axis of the recess (7) and on the clamp body (6) forms two resilient clamping jaws (8, 9) which when clamped together secure the implant pin (1) in the clamp body (6), the clamp body (6) and/or the implant pin (1) incorporating relieving recesses (6.3, 6.4, 1.3, 1.4) in the region of the exit cross-sections (1.1, 1.2) of the implant pin (1) from the clamp body (6), in order to prevent the clamp body (6) from exerting a notching and squeezing action on the implant pin (1) during deflections of the implant pin (1).

8. Clamp arrangement (1, 6) according to claim 7,
characterised in that
the relieving recesses are disposed on the clamp body (6) and are formed by broken or rounded edges (6.3, 6.4) at the interface between the external surfaces (6.1, 6.2) and the recess (7) of the clamp body (6).

9. Clamp arrangement (1, 6) according to claim 8,
characterised in that
the relieving recesses are disposed on the implant pin (1) and are in each case formed by an all-round groove (1.3, 1.4) which preferably possesses rounded edges and corners.

10. Clamp arrangement (1, 6) according to claims 1 and 7,
characterised in that
the relieving recesses are formed by the recessed areas disposed in the region surrounding the exit cross-sections.

11. Internal fixator with a clamp arrangement (1, 6) according to at least one of claims 1 to 10.

12. External fixator with a clamp arrangement (1, 6) according to at least one of claims 1 to 10.

## Revendications

1. Agencement de serrage (1, 6) pour un fixateur orthopédique, comprenant une broche d'implant (1) pouvant être fixée dans un os, et un corps de pince (6) comportant un évidement (7) dont la surface génératrice joint deux surfaces extérieures opposées (6.1, 6.2) du corps de pince (1) l'une à l'autre et reçoit directement la broche d'implant (1), le corps de pince comprenant une fente partant de l'évidement (7) et dont le prolongement imaginaire contient l'axe longitudinal de l'évidement (7), la fente définissant deux mors élastiques (8, 9) dans le corps de pince (6), où la broche d'implant (1) est fixée dans le corps de pince (6) lorsque les mors sont serrés l'un sur l'autre, l'une des surfaces tournées l'une vers l'autre, de l'évidement (7) du corps de pince (6) et de la broche d'implant (1), comprenant des zones en retrait (6.7, 6.8).

2. Agencement de serrage (1, 6) selon la revendication 1, caractérisé en ce que la surface comportant les zones en retrait est la paroi de l'évidement (7) du corps de pince (6).

3. Agencement de serrage (1, 6) selon la revendication 1, caractérisé en ce que la surface comportant les zones en retrait est la surface extérieure de la broche d'implant (1).

4. Agencement de serrage (1, 6) selon la revendication 1, caractérisé en ce que les zones en retrait sont formées par un évidement hélicoïdal (6, 7).

5. Agencement de serrage (1, 6) selon la revendication 1, caractérisé en ce que les zones en retrait sont formées par plusieurs rainures (6.8) s'étendant de préférence perpendiculairement à l'axe longitudinal de l'évidement (7) ou de la broche d'implant (1).

6. Agencement de serrage (1, 6) selon la revendication 4 ou 5, caractérisé en ce que chacune des zones en retrait, vue en coupe suivant l'axe longitudinal de l'évidement (7) ou de la broche d'implant (1), forme un contour ondulé, trapézoïdal, rectangulaire ou en forme de queue d'aronde, le cas échéant pourvu de bords et d'angles arrondis.

7. Agencement de serrage (1, 6) pour un fixateur orthopédique, comprenant une broche d'implant (1) pouvant être fixée dans un os, et un corps de pince (6) comportant un évidement (7) dont la surface génératrice joint deux surfaces extérieures opposées (6.1, 6.2) du corps de pince (6) l'une à l'autre et reçoit directement la broche d'implant (1), le corps de pince comprenant une fente partant de l'évidement (7) et dont le prolongement imaginaire contient l'axe longitudinal de l'évidement (7), la fente définissant deux mors élastiques (8, 9) dans le corps de pince (6), où la broche d'implant (1) est fixée dans le corps de pince (6) lorsque les mors sont serrés l'un sur l'autre, le corps de pince (6) et/ou la broche d'implant (1) présentant, au niveau des sections de sortie (1.1, 1.2) de la broche d'implant (1) sortant du corps de pince (6), des évidements de réduction des contraintes (6.3, 6.4; 1.3, 1.4), afin d'éviter, en cas de courbure de la broche d'implant (1), un effet d'entaille ou de compression exercé par le corps de pince (6) sur la broche d'implant (1).

8. Agencement de serrage (1, 6) selon la revendication 7, caractérisé en ce que les évidements de diminution des contraintes sont aménagés dans le corps de pince (6) et sont constitués par des bords chanfreinés ou arrondis (6.3, 6.4) situés au niveau de la jonction entre les surfaces extérieures (6.1, 6.2) et l'évidement (7) du corps de pince (6).

9. Agencement de serrage (1, 6) selon la revendication 8, caractérisé en ce que les évidements de diminution des contraintes sont aménagés dans la broche d'implant (1) et sont constitués respectivement par une gorge périphérique (1.3, 1.4) présentant de préférence des bords arrondis.

10. Agencement de serrage (1, 6) selon les revendications 1 et 7, caractérisé en ce que les évidements de diminution des contraintes sont formés par les zones en retrait situées à proximité des sections de sortie.

11. Fixateur interne comprenant un agencement de serrage (1, 6) selon l'une au moins des revendications 1 à 10.

12. Fixateur externe comprenant un agencement de serrage (1, 6) selon l'une au moins des revendications 1 à 10.
